# EUROPÄISCHE PATENTANMELDUNG

(11) **EP 0 969 084 A1**
(43) Veröffentlichungstag der Anmeldung: **05.01.2000**
(21) Anmeldenummer: 99110440.7
(22) Anmeldetag: 29.05.1999
(51) Int. Cl.: C12M 1/107

(54) **Reaktor zur Vergärung biogener Stoffe**

(30) Priorität: 04.06.1998 DE 19824867
(71) Anmelder: DEUTSCHE BABCOCK ANLAGEN GMBH, 46049 Oberhausen (DE)
(72) Erfinder: Dohmann, Joachim, Dr., 46149 Oberhausen (DE); Mian, Iqbal Muhammad, 44149 Dortmund (DE)
(74) Vertreter: Radünz, Ingo, Dipl.-Ing.

(57) **Zusammenfassung**

Reaktor zur Vergärung biogener Stoffe besteht aus einem Behälter (1), der eine die biogenen Stoffe und Biomasse enthaltende Suspension aufnimmt, der mit einer Einlaßöffnung (2) für die Suspension und mit mehreren Auslaßöffnungen (4, 5) für die Abführung der Reaktionsprodukte der Vergärung versehen ist und der Mittel zur Erzeugung einer Umlaufströmung in der Suspension enthält. In dem Behälter (1) sind ein oder mehrere oben und unten offene, in die Suspension eingetauchte senkrechte Schächte (6) angeordnet. An den Behälter (1) ist eine mit mindestens einer Umwälzpumpe (10) versehene Umlaufleitung (9) angeschlossen, über die der mit der Suspension gefüllte Innenraum des Behälters (1) mit dem Innenraum des oder der Schächte (6) verbunden ist.

## Beschreibung

Die Erfindung betrifft einen Reaktor zur Vergärung biogener Stoffe mit den Merkmalen des Oberbegriffes des Patentanspruches 1.

Bei bekannten Vergärungsverfahren (Biologische Abfallbehandlung / K. J. Thomé-Kozmiensky. - Berlin : EF-Verlag für Energie und Umwelttechnik, 1995, Seiten 418 - 421) wird ein Biomasse enthaltendes Material zunächst vorbehandelt und anschließend der Vergärung zugeführt. Die bei der Vergärung entstehenden Stoffströme bestehen zum einen aus einem methanhaltigen Gas, zum anderen aus einem meist als Suspension vorliegenden Gärrest. Die Reaktionsprodukte werden aus dem Gärreaktor ausgeschleust und einer Nachbehandlung oder einer Verwendung zugeführt. Die Vorbehandlung umfaßt in der Regel eine Zerkleinerung des Gutes, die Entfernung von Störstoffen und die Herstellung einer Suspension mit einem für die anschließende Vergärung günstigen Feststoff- und Wassergehalt.

Das vorbehandelte Gut wird einem Reaktor zugeführt, in dem ein biologischer Abbau der organischen Bestandteile stattfindet. An die Reaktionsführung innerhalb des Reaktors sind verschiedene Anforderungen zu stellen. So siedeln sich die für den Abbau erforderlichen Mikroorganismen auf den Oberflächen des Gutes an oder bilden flockige Agglomerate. Die Reaktionsprodukte der Mikroorganismen werden auf die wäßrige Phase der Suspension übertragen, was sich z. B. in der Änderung der lokalen pH-Werte bemerkbar macht. Eine fehlende oder gehemmte Übertragungsmöglichkeit führt zu einer Minderung der Wachstumsrate der Mikroorganismen oder im Extremfall zu einer Desaktivierung des Stoffwechsels der Mikroorganismen. Daraus leitet sich als eine Anforderung an die Strömungsführung innerhalb des Reaktors ab, daß die wäßrige Phase eine geringfügige Drift relativ zur Feststoffphase der Biosuspension besitzen muß. Dadurch wird der im allgemeinen unzureichende diffusive Stofftransport von einem konvektiven Transport überlagert.

In dem bekannten Vergärungsverfahren (Biologische Abfallbehandlung / K. J. Thomé-Kozmiensky. - Berlin : EF-Verlag für Energie und Umwelttechnik, 1995, Seiten 418 - 421) wird versucht, diese Driftbewegung durch Einbringung von Produktgas in den unteren Teil des Reaktors zu erzeugen. Das Produktgas wandert in Form suspendierter Blasen zur freien Oberfläche der Suspension. Bei geringen Feststoffgehalten der Suspension setzt hierdurch eine makroskopische Mischbewegung ein. Die entstehenden Strömungsfelder sind vergleichbar mit denen in sogenannten Blasensäulen. Vorteilhaft an dieser Art des pneumatischen Rührens ist die geringe mechanische Beanspruchung des Gutes, die die Vitalität der Mikroorganismen nicht beeinträchtigt. Bei hohen Feststoffkonzentrationen versagt dieses Mischverfahren, so daß bei der pneumatischen Mischung der Feststoffgehalt der Suspension zu begrenzen ist. Das Mischverfahren stellt somit in diesem Sinne eine Restriktion der Verfahrensparameter dar.

Pneumatische Mischverfahren durch Mischen mittels mechanischer Mischelemente zu ersetzen, ist wenig erfolgreich, da durch faserige Bestandteile des zu behandelnden Gutes eine Funktionsbeeinträchtigung der Rührorgane herbeigeführt wird. Zum anderen besteht die Gefahr einer Minderung der Wachstumsraten durch lokales und temporäres Überschreiten zulässiger Schubspannungen, die in der Regel zu einer Vitalitätseinbuße der Mikroorganismen führt.

Eine weitere Anforderung an die Reaktionsführung betrifft das Verweilzeitverhalten der abzubauenden Stoffe innerhalb des Reaktors. Die Aufenthaltsdauer dieser Stoffe muß hinreichend lang sein, damit innerhalb der zur Verfügung stehenden Zeitspanne die Abbaureaktion in ausreichendem Umfang ablaufen kann. Das frisch eingebrachte Gut wird in bekannten Anlagen mit noch unvollständig abgebautem Gut vermischt, was zu einer Impfung des Inputstromes führt und kurze Abbauzeiten bewirkt. Bekannte Reaktoren werden entweder zweistufig ausgeführt, oder aber besitzen innerhalb eines Reaktors verschiedene Zonen mit unterschiedlicher Verweilzeitcharakteristik.

Insbesondere bei größeren Feststoffkonzentrationen in der Suspension verändert die Eigenschaft des Gutes, während des Abbauprozeßes Agglomerate zu bilden, die rheologischen Eigenschaften der Suspension. Die Agglomerationsbildung führt zunächst zu einem Anstieg der Viskosität der Suspension. Bei weiterer Agglomeration wird die Ausbildung einer Fließgrenze der Suspension beobachtet. Strömungsbewegungen treten erst dann auf, wenn die treibenden Kräfte kritische Werte der lokalen Schubspannung übersteigen. Diese Fließgrenze hat ihre anschauliche Ursache in der Ausbildung räumlicher Netzwerke, die aus der Vernetzung der Feststoffbestandteile der Suspension entstehen. Die Kontaktstellen sind imstande, mechanische Spannungen zu übertragen, ohne daß ihr Verbund gelöst wird. Erst bei der Überschreitung kritischer Spannungen werden benachbarte Partikel voneinander getrennt. Dies führt in der Praxis dazu, daß Teilvolumina der Reaktoren Suspensionen enthalten, die aufgrund der Agglomeration erstarren und keine makroskopischen Fließbewegungen zeigen. In dichteren Netzwerken ist die Abfuhr der Reaktionsprodukte über die flüssige Phase nicht zu erreichen. Die Suspension innerhalb dieser Volumina verdichtet sich aufgrund verschiedener Effekte weiter, was zu einer fortschreitenden Erhöhung der lokalen Fließgrenze führt. Dies zieht eine Blockierung dieser Teilvolumina nach sich. Stoffaustauschvorgänge finden innerhalb dieser Bereiche nur eingeschränkt und unzureichend statt. Dies hat eine Reduktion des effektiven Reaktorvolumens zur Folge, weshalb die mittlere Aufenthaltsdauer des Gutes abnimmt. Der mit dem Reaktor erreichbare Gesamtumsatzgrad nimmt damit unzureichend niedrige Werte an. Praktisch handelt es sich um ein Versagen des Bioreaktors.

Durch die Einbringung von rückgeführtem Produktgas in den Reaktor können die genannten blockierten Bereiche nicht remobilisiert werden. Dabei werden der Suspension Schubspannungen aufgeprägt, die aber unterhalb der Fließspannungen liegen. In diesem Fall wird die lokale Bildung von Kanälen innerhalb der Biomasse beobachtet, durch die das eingebrachte Produktgas ohne die Erzeugung makroskopischer Misch- und Fließbewegungen strömt. In bekannten Reaktoren kann diese Art von Betriebsstörung oftmals nur durch die Entleerung des Reaktors und den Austausch der Suspension erreicht werden.

Der Erfindung liegt die Aufgabe zugrunde, den gattungsgemäßen Reaktor derart zu gestalten, daß eine Suspension mit einem erhöhten Feststoffgehalt verarbeitet werden kann.

Diese Aufgabe wird bei einem gattungsgemäßen Reaktor erfindungsgemäß durch die kennzeichnenden Merkmale des Patentanspruches 1 gelöst. Vorteilhafte Ausgestaltungen der Erfindung sind Gegenstand der Unteransprüche.

Durch die Anordnung von Schächte und die Rückführung eines Teilstromes der Suspension werden in dem erfindungsgemäßen Reaktor mindestens zwei räumlich voneinander getrennte Zonen mit unterschiedlich orientierter Durchströmung gebildet. In den Schächten wird eine vorzugsweise aufwärts gerichtete Strömungsgeschwindigkeit eingestellt, die z. B. doppelt so groß ist wie die abwärts gerichtete Strömungsgeschwindigkeit in dem Mantelraum des Behälters außerhalb der Schächte. Die Rückspeisung der umgepumpten Suspension sorgt zudem durch eine Injektorwirkung für ein Ansaugen von Suspension, wodurch es zu einer intensiven Rückvermischung der Suspension kommt. Während des Durchströmens durch die Schächte wird die Suspension mechanisch beansprucht, wodurch einer Agglomeration entgegengewirkt wird. In dem Mantelraum außerhalb der Schächte findet bei verringerter Strömungsgeschwindigkeit keine Mischung statt, und es besteht eine ausreichende Zeitspanne zur Regeneration der Mikroorganismen und zum Abbau der biogenen Stoffe. Diese Strömungs- und Verweilcharakteristik erlaubt es, daß in dem erfindungsgemäßen Reaktor Suspensionen mit höheren Feststoffgehalten umgesetzt werden können als dies mit herkömmlichen, bekannten Reaktoren der Fall war.

Ein Ausführungsbeispiel der Erfindung ist in der Zeichnung dargestellt und wird im folgenden näher erläutert. Es zeigen:
- Fig. 1: den Längsschnitt durch einen Reaktor zur Vergärung von biogenen Stoffen und
- Fig. 2: den Querschnitt zu Fig. 1.

Der Reaktor besteht aus einem geschlossenen, vorzugsweise stehenden, zylindrischen Behälter 1. Der Behälter 1 ist mit einer Einlaßöffnung 2 versehen, deren Position beliebig ist und beispielsweise im unteren oder oberen Teil des Behälters 1 angeordnet sein kann. Über diese Einlaßöffnung 2 werden die umzusetzenden, biogenen Stoffe in Form einer angemaischten Suspension mit einem vorbestimmten Massenanteil an Trockensubstanz in den Behälter 1 eingespeist. Der Suspension kann Prozeßwasser zugesetzt werden, falls die Stoffe nicht von sich aus genügend Wasser zur Herstellung der Suspension enthalten. Der Flüssigkeitsspiegel, bis zu dem der Behälter 1 mit der Suspension gefüllt ist, ist durch die Linie 3 angedeutet.

Im dem unteren, konisch ausgebildeten Teil des Behälters 1 ist eine Auslaßöffnung 4 für die Entfernung der umgesetzten Biomasse angeordnet. In dem oberen Teil des Behälters 1 ist eine Auslaßöffnung 5 für die Abführung des bei der Umsetzung der Suspension mit den Mikroorganismen gebildeten Biogases.

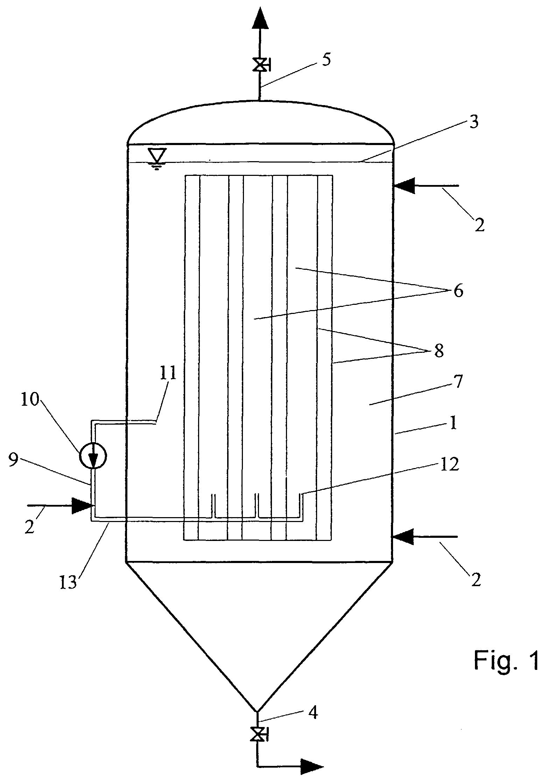

Innerhalb des Behälters 1 sind oben und unten offene senkrechte Schächte 6 angeordnet, die bei gefülltem Behälter 1 in die Suspension eingetaucht sind und gemäß Fig. 1 bis nahe an den Flüssigkeitsspiegel 3 heran reichen. Die Oberkanten der Schächte 6 können auch abweichend davon über den Flüssigkeitsspiegel 3 hinaus ragen. Die Schächte 6 sind in der Mitte des Behälters 1 vorgesehen, so daß zwischen den Schächten 6 und der Wandung des Behälters 1 ein Mantelraum 7 gebildet ist.

Jeder Schacht 6 ist durch ebene Wände 8 gebildet, die unter einem Winkel aneinander stoßen. Vorzugsweise bilden die Schächte 6 im Querschnitt ein regelmäßiges Vieleck, vorzugsweise ein Sechseck. Die Schächte 6 liegen dicht nebeneinander, wobei einzelne Wände 8 zu zwei benachbarten Schächten 6 gehören. Auf diese Weise entsteht ein sich selbst stabilisierender Wabenkörper. Ebenfalls geeignet sind ein oder mehrere Schächte, deren Querschnitt kreisförmig ist.

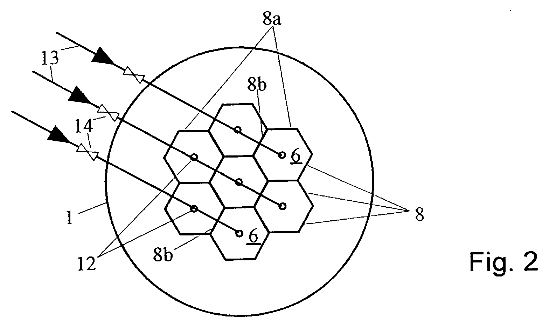

Einzelne der ebenen Wände 8, die in Fig. 2 mit 8a und 8b bezeichnet sind, fluchten miteinander. Diese Wände 8a, 8b werden über die übrigen Wände 8 hinaus verlängert und können durch ein gemeinsames Tragwerk verbunden werden, das an der Wandung des Behälters 1 befestigt wird. Eine derartige Konstruktion kann mit wenigen Befestigungspunkten als hängende Konstruktion ausgeführt werden. Diese Anordnung eignet sich auch zur Nachrüstung bereits errichteter Reaktoren. Hilfsweise können auch einige der Wände 8 über das untere Ende der Schächte 6 hinaus verlängert werden. Diese Konstruktion kann dann auf den Verlängerungen stehen, ohne daß die von unten in die Konstruktion eintretende freie Strömung übermäßig behindert würde.

Der Behälter 1 ist mit einem Flüssigskeitsumlauf versehen, der eine Umlaufleitung 9 umfaßt. In der Umlaufleitung 9 ist eine Umwälzpumpe 10 angeordnet, die vorzugsweise als Verdrängerpumpe, zum Beispiel als Membranpumpe ausgebildet ist. Der oder die Ansaugpunkte 11 der Umlaufleitung 9 liegen innerhalb des Mantelraumes 7 der Behälters 1. Die Rückspeisungspunkte 12 befinden sich in einer Ebene unterhalb der Ansaugpunkte 11 und innerhalb der Schächte 6. Die Ansaugpunkte 11 und die Rückspeisungspunkte 12 sind auf diese Weise voneinander getrennt, was eine Kurzschlußströmung verhindert.

Die Umlaufleitung 9 verzweigt sich stromabwärts von der Umwälzpumpe 10 in mehrere Lanzen 13, wobei eine Lanze 13 zu einem oder zu einer Gruppe von Schächten 6 geführt ist. Durch die Anordnung von Ventilen 14 in den Lanzen 13 können die Lanzen 13 einzeln zu- oder abgeschaltet werden.

Durch den Betrieb der außerhalb des Behälters 1 angeordneten Umwälzpumpe 10 wird in dem Reaktor eine Strömungsbewegung eingehalten, bei der die Schächte 6 von unten nach oben und der Mantelraum 7 von oben nach unten durchströmt ist. Die Suspension wird im unteren Bereich der Schächte 6 eingebracht. Dabei kann zusätzlich eine Injektorwirkung ausgenutzt werden, um die Suspension aus dem Raum unterhalb der Schächte 6 anzusaugen und gemeinsam mit dem durch die Umwälzpumpe 10 durchgesetzten Suspensionsstrom durch die Schächte 6 zu fördern. Durch die Vereinigung des gepumpten Suspensionstromes und des frei angesaugtem Stromes kommt es zu einer intensiven Rückvermischung der Suspension. Bei der Verwendung einer zwangsfördernden Verdrängerpumpen ist der Volumenstrom, der durch die Schächte 6 durchgesetzt wird, weitgehend unabhängig von den rheologischen Eigenschaften der Suspension. Liegt eine ausgeprägte Fließgrenze vor, so kann diese überwunden werden. Durch die mechanische Beanspruchung kann bei der Durchströmung der Schächte 6 die Fließgrenze herabgesetzt werden. Dies erlaubt in dem Reaktor den Umsatz von Suspensionen mit erhöhten Feststoffgehalten.

Die aufwärtige Strömungsgeschwindigkeit in den Schächten 6 ist niedrig, aber oberhalb der Sedimentationsgeschwindigkeit der in der Suspension enthaltenen Feststoffe von z. B. 0,05 m/s, zu wählen. Dadurch kommt es zur Ausbildung einer die Vermischung begünstigenden Wirbelschicht. Die Strömungsgeschwindigkeit ist aber ausreichend groß zu wählen, damit die Feststoffe der Suspension am oberen Ende der Schächte 6 ausgetragen werden können. In dem Mantelraum 7 des Reaktors entsteht eine abwärts gerichtete Strömung. Dieser Fließbewegung ist die Sedimentationsbewegung der Feststoffe überlagert.

Die höchsten auftretenden Geschwindigkeiten der Suspension treten, wenn man von den Umlauf- und Förderleitungen absieht, die mit den Umwälz- und Förderpumpen verbunden sind, in dem aufwärtsgerichteten Bereich der Schächte 6 auf. Es ist vorteilhaft, wenn die aufwärtsgerichtete Strömungsgeschwindigkeit größer ist als das Zweifache (z. B. Vierfache) der abwärts gerichteten Strömungsgeschwindigkeit. Dies wirkt sich günstig auf die während des Suspensionsumlaufs auftretenden und zur Überwindung der Fließgrenzen erforderlichen Schubspannungen aus. Zum anderen sichert dies eine geringe Baugröße der Schächte 6.

Der erfindungsgemäße Reaktor besitzt ein in weitem Umfang regelbares Verweilzeitverhalten. Je nach Art der Suspensionsumwälzung sind mit dem Reaktor mehrere Betriebsweisen möglich:

### 1. Kontinuierlicher Betrieb:

Der kontinuierliche Betrieb ist gekennzeichnet durch einen stetigen, ununterbrochenen, internen Flüssigkeitsumlauf. Dieser Flüssigkeitsumlauf ist von Vorteil, wenn die rheologischen Eigenschaften der Suspension durch das Auftreten einer Fließgrenze geprägt sind, und diese Fließgrenze überschritten wird. Durch die Anordnung der Schächte 6 in dem Reaktor und durch die aufgrund der Rückführung eines Teilstromes der Suspension erzeugten Strömungsfelder innerhalb des Reaktors wird dieser Eigenart entgegengewirkt. In den Schächten 6 wird durch eine erhöhte Strömungsgeschwindigkeit die Fließgrenze der Suspension herabgesetzt, so daß es zu keiner Blockierung kommen kann. Andererseits ist in anderen Teilbereichen der Strömung, nämlich in dem Mantelraum 7, die mechanische Beanspruchung der Suspension niedriger ist als die für die Zerstörung von Mikroorganismen kritischen Spannungen und es besteht die Voraussetzung für eine ausreichende Reaktionszeit.

### 2. Intermittierender Betrieb:

Vorteilhaft kann der intermittierende Betrieb der Umwälzung sein. Die Zeitkonstanten, die mit der Bildung der räumlichen Netzwerke im Zusammenhang stehen und jene, die mit der temporären Zerstörung der Verknüpfungspunkte verbunden sind, können sich um einige Größenordnungen unterscheiden. Insbesondere bei geringen Feststoffkonzentrationen vollzieht sich ein Aufbau der Netzwerke langsamer als ein möglicher Abbau. In diesem Fall reicht die mit einer gelegentlichen Rückführung der Suspension eingetragene Energie aus, um die Fließgrenzen der Suspension niedrig zu halten. Aber auch bei hohen Feststoffgehalten kann ein intermittierender Betrieb vorteilhaft sein, da es nicht primäres Ziel ist, das rheologische Verhalten der Suspension einzustellen, sondern Reaktionsprodukte abzuführen und eine angemessene Verweilzeitverteilung zu erreichen. Entscheidend ist, daß zu gewünschten Zeitpunkten eine makroskopische Vermischung möglich ist.

### 3. Pausierender Betrieb

Bei dem pausierenden Betrieb wird zeitweilig ein einzelner Schacht 6 oder eine Gruppe von Schächten 6 durch ein Schließen der Ventile 14 in den entsprechenden Lanzen 13 von der Durchströmung ausgeschlossen. Das von dem oder den Schächten 6 umschlossene Volumen dient als Reservoir für Suspension. Auf diese Weise können bei Teillastbetrieb die günstigsten Strömungsbedingungen in allen Bereichen des Reaktors sichergestellt werden.

## Patentansprüche

1. Reaktor zur Vergärung biogener Stoffe bestehend aus einem Behälter (1), der eine die biogenen Stoffe und Biomasse enthaltende Suspension aufnimmt, der mit einer Einlaßöffnung (2) für die Suspension und mit mehreren Auslaßöffnungen (4, 5) für die Abführung der Reaktionsprodukte der Vergärung versehen ist und der Mittel zur Erzeugung einer Umlaufströmung in der Suspension enthält, dadurch gekennzeichnet, daß in dem Behälter (1) ein oder mehrere oben und unten offene, in die Suspension eingetauchte senkrechte Schächte (6) angeordnet sind und daß an den Behälter (1) eine mit mindestens einer Umwälzpumpe (10) versehene Umlaufleitung (9) angeschlossen ist, über die der mit der Suspension gefüllte Innenraum des Behälters (1) mit dem Innenraum des oder der Schächte (6) verbunden ist.

2. Reaktor nach Anspruch 1, dadurch gekennzeichnet, daß die Rückspeisungspunkte (12) der Umlaufleitung (9) unterhalb der Ansaugpunkte (11) der Umlaufleitung (9) und innerhalb der Schächte (6) vorgesehen sind.

3. Reaktor nach Anspruch 1 oder 2, dadurch gekennzeichnet, daß der Schacht oder die Schächte (6) in der Mitte des Behälters (1) angeordnet sind.

4. Reaktor nach einem der Ansprüche 1 bis 3, dadurch gekennzeichnet, daß der Schacht oder die Schächte (6) durch ebene Wände (8) begrenzt sind.

5. Reaktor nach Anspruch 4, dadurch gekennzeichnet, daß der Schacht oder die Schächte (6) im Querschnitt als regelmäßige Vielecke, vorzugsweise als regelmäßiges Sechseck ausgebildet sind.

6. Reaktor nach einem der Ansprüche 1 bis 3, dadurch gekennzeichnet, daß der Schacht oder die Schächte (6) mit kreisförmigem Querschnitt ausgebildet sind.

7. Reaktor nach Anspruch 4 oder 5, dadurch gekennzeichnet, daß die Schächte (6) als im Querschnitt sechseckige Waben ausgebildet sind.

8. Reaktor nach Anspruch 4, 5 oder 7, dadurch gekennzeichnet, daß einzelne Wände (8a, 8b) mehrerer Schächte (6) miteinander fluchtend angeordnet sind, die übrigen Wände (8) überragen und durch ein gemeinsames Tragwerk miteinander verbunden sind, das an dem Behälter (1) oder dessen Abdeckung befestigt ist.

9. Reaktor nach einem der Ansprüche 1 bis 8, dadurch gekennzeichnet, daß das obere Ende der Schächte (6) unterhalb des freien Flüssigkeitsspiegels (3) innerhalb des Behälters (1) endet.

10. Reaktor nach einem der Ansprüche 1 bis 8, dadurch gekennzeichnet, daß das obere Ende der Schächte (6) aus dem freien Flüssigkeitsspiegel (3) innerhalb des Behälters (1) herausragt.

11. Reaktor nach einem der Ansprüche 1 bis 10, dadurch gekennzeichnet, daß die Umwälzpumpe (10) als Verdrängerpumpe ausgebildet ist.

12. Reaktor nach einem der Ansprüche 1 bis 10, dadurch gekennzeichnet, daß die Umlaufleitung (9) in mehrere Lanzen (13) verzweigt ist, an deren Enden die Rückspeisungspunkte (12) vorgesehen sind und daß jede Lanze (13) zu einem Schacht (6) oder einer Gruppe von Schächten (6) geführt ist.

13. Reaktor nach Anspruch 12, dadurch gekennzeichnet, daß die Lanzen (13) einzeln abschaltbar sind.
